# EUROPÄISCHE PATENTSCHRIFT

(11) **EP 0 470 290 B1**
(45) Veröffentlichungstag und Bekanntmachung des Hinweises auf die Patenterteilung: **23.10.1996**
(21) Anmeldenummer: 90115421.1
(22) Anmeldetag: 10.08.1990
(51) Int. Cl.: C12M 1/40

(54) **Elektrochemisch-enzymatischer Sensor**
Electrochemical enzymatic sensor
Senseur électrochimique enzymatique

(43) Veröffentlichungstag der Anmeldung: 12.02.1992
(73) Patentinhaber: SIEMENS AKTIENGESELLSCHAFT, 80333 München (DE)
(72) Erfinder: Preidel, Walter, Dr. Dipl.-Chem., D-8520 Erlangen (DE)

(56) Entgegenhaltungen:
- EP-A- 0 127 958
- EP-A- 0 136 362
- EP-A- 0 359 831
- GB-A- 1 167 317
- US-A- 4 655 880

## Beschreibung

Die Erfindung betrifft einen elektrochemisch-enzymatischen Sensor zur Bestimmung von Substanzen, insbesondere Glucose, in Körperflüssigkeiten sowie eine implantierbare Sensoranordnung.

Die Messung der Glucosekonzentration im Blut (mittels eines implantierbaren Sensors) ist beispielsweise bei der Diabetestherapie - zur Steuerung der Insulindosierung mittels einer implantierten Pumpe - erforderlich. Dazu wird ein Sensor benötigt, der spezifisch auf Glucose anspricht, eine gute Langzeitstabilität aufweist und biokompatibel ist. Ein derartiger Glucosesensor wäre zudem auch bei klinischen Untersuchungen für die Glucosemessung und bei Glucosetoleranztests einsetzbar, d.h. er muß nicht notwendigerweise implantiert werden.

Die Bestimmung von Glucose im Blut kann mit Enzymsensoren erfolgen, wobei im allgemeinen Glucoseoxidase als Enzym dient (siehe dazu: J. G. Schindler "Bioelektrochemische Membranelektroden", Verlag Walter de Gruyter, Berlin - New York, 1983, Seiten 211 ff.). Der Verwendung von enzymatischen Glucosesensoren zur Diabetestherapie steht bislang allerdings die mangelnde Langzeitstabilität der Sensoren entgegen. Verschiedene Maßnahmen, das Enzym zu stabilisieren, beispielsweise durch Einschluß in eine Matrix, chemische Bindung oder Adsorption, führen zwar zu einer Verbesserung der Stabilität, aber auch zu einer Verminderung der Selektivität für Glucose. Die bislang erreichbare Langzeitstabilität von Enzymsensoren beträgt deshalb im allgemeinen lediglich bis zu 30 Tagen in vitro bzw. bis zu 14 Tagen in vivo, obwohl auch bereits über eine Implantationsdauer von ca. 100 Tagen berichtet wurde (siehe dazu: "Nachr. Chem. Tech. Lab.", Bd. 38 (1990), Seiten 868 und 869).

Enzymatische Glucosesensoren arbeiten nach folgendem Prinzip: 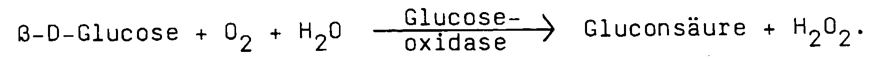 Dabei wird - als Maß für die Glucosekonzentration - entweder die Verarmung an Sauerstoff (O₂) oder die Anreicherung an Wasserstoffperoxid (H₂O₂) bestimmt. Die Messung erfolgt potentiometrisch oder amperometrisch, wobei eine Meßelektrode aus Edelmetall, im allgemeinen Platin, verwendet wird. Die mangelnde Langzeitstabilität des Enzyms, d.h. die Ursache für die Alterung, dürfte dabei in einer Beeinflussung (des Enzyms) durch das H₂O₂ oder durch die Meßelektrode bzw. durch Zersetzung des Enzyms an der Meßelektrode gebildete Produkte begründet sein.

Aus der US-PS 4 655 880 ist ein Sensor bekannt, bei dem als Enzym eine Oxidase verwendet wird. Als Material für die Arbeitselektrode dienen Edelmetalle, wobei bevorzugte Materialien Gold, Platin, Silber und Kohlenstoff sind, d.h. also elektrokatalytisch aktive Materialien. Bei einer Vorrichtung zur polarographischen Analyse, die aus der GB-PS 1 167 317 bekannt ist, wird an der Arbeitselektrode Wasserstoffperoxid umgesetzt. Das Elektrodenmaterial ist ein elektrokatalytisch aktives Material, und zwar Platin oder pyrolytischer Graphit. Im Falle von pyrolytischem Graphit wird dem Elektrolyt dazu Palladiumpulver oder ein Palladiumsalz zugesetzt, so daß sich die Pyrographitelektrode wie eine Elektrode aus Palladium verhält. Bei weiteren bekannten Sensoren (siehe EP-OS 0 359 831 und EP-OS 0 127 958) kann in den Elektroden Kohlenstoff Verwendung finden. Das wirksame Agens der Elektroden ist - neben einem Enzym - jedoch ein Elektronenakzeptor bzw. ein Mediator.

Aufgabe der Erfindung ist es, einen elektrochemisch-enzymatischen Sensor der eingangs genannten Art in der Weise auszugestalten, daß keine Beeinflussung des Enzyms durch die Meßelektrode (Sensorelektrode) erfolgt und somit eine gute Langzeitstabilität gegeben ist.

Dies wird erfindungsgemäß durch einen Sensor mit folgenden Merkmalen erreicht:
- eine Sensorelektrode aus elektrokatalytisch inaktivem Kohlenstoff in Form von Glaskohlenstoff, Pyrographit, gesputtertem Kohlenstoff, gesputtertem Graphit oder amorphem wasserstoffhaltigem Kohlenstoff, an der nur Sauerstoff umgesetzt wird
- eine Gegenelektrode
- eine Bezugselektrode
- eine vor der Sensorelektrode befindliche Enzym enthaltende Schicht und
- eine die Enzymschicht zur Körperflüssigkeit hin abdeckende und das Enzym zurückhaltende Membran aus biokompatiblem, hydrophilem, sauerstoffdurchlässigem Material.

Dem elektrochemisch-enzymatischen Sensor nach der Erfindung liegt das Prinzip eines Sauerstoffsensors zugrunde, d.h. es wird die Änderung des Sauerstoffgehaltes bestimmt; diese Änderung besteht vorwiegend in einer Abreicherung (an O₂). Durch die Kombination der Enzymreaktion mit einem O₂-Sensor, der das Enzym nicht beeinflußt und somit die Enzymaktivität nicht verändert, wird eine hohe Langzeitstabilität erreicht. Beim erfindungsgemäßen Sensor besteht nämlich die Meß- bzw. Sensorelektrode aus elektrokatalytisch inaktivem Kohlenstoff. An einer derartigen Elektrode wird aber nur Sauerstoff umgesetzt, d.h. reduziert, andere Reaktionen sind dagegen stark gehemmt, und auch das Enzym reagiert nicht, d.h. es wird elektrochemisch nicht verändert.

Beim Sensor nach der Erfindung findet auch keine Vergiftung der Meßelektrode durch Substanzen statt, die möglicherweise durch die Membran und die Enzymschicht zur Elektrode diffundieren. Da somit keine schädlichen Elektrodenreaktionen ablaufen, erfolgt auch keine Verminderung der Enzymaktivität durch Reaktionsprodukte derartiger Reaktionen. Dieser Sensor erfordert ferner keine zusätzlichen Substanzen zur Elektronenübertragung, wie Ferrocen und Tetracyanochinodimethan, weil an der Sensorelektrode eine Reduktion von Sauerstoff erfolgt.

Der erfindungsgemäße Sensor dient vorzugsweise zur Bestimmung von Glucose (mit Glucoseoxidase). Das diesem Sensor zugrundeliegende Prinzip der Kombination eines Sauerstoffsensors mit einer Enzymreaktion, die Sauerstoff erzeugt oder verbraucht, läßt sich aber auch zur Bestimmung anderer Substanzen verwenden. Beispiele dafür sind:
- (verschiedene) Zucker mit entsprechender Oxidase, wie Galactose mit Galactoseoxidase
- Lactat mit Lactatoxidase
- Methanol bzw. Ethanol mit Alkoholoxidase
- Cholesterin mit Cholesterinoxidase
- Harnsäure mit Uricase
- Ascorbinsäure mit Ascorbatoxidase
- Pyruvat mit Pyruvatoxidase
- Adenosintriphosphat mit Glucoseoxidase/Hexokinase und
- Wasserstoffperoxid mit Katalase.

Bei der letztgenannten Reaktion, der H₂O₂-Bestimmung, erfolgt im übrigen die Bildung von Sauerstoff, d.h. eine Anreicherung.

Beim erfindungsgemäßen Sensor kann das Enzym sowohl in gelöster als auch in immobilisierter Form eingesetzt werden. Bei der Immobilisierung, die insbesondere durch Vernetzung, beispielsweise - zusammen mit Rinderserumalbumin - mittels Glutaraldehyd, oder durch Einschluß in eine Matrix, beispielsweise aus Polyacrylamid, erfolgen kann, geht im allgemeinen allerdings ein Teil der Enzymaktivität verloren, wodurch die Lebensdauer eingeschränkt wird. Um eine höhere Langzeitstabilität zu erreichen, kann deshalb dem Enzym vorteilhaft Katalase zugesetzt werden. Dadurch soll die Anreicherung von H₂O₂ vermieden werden, welches das Enzym möglicherweise schädigt. Außerdem kann das Enzym auch in regelmäßigen Abständen ausgetauscht werden.

Der Sensor nach der Erfindung weist eine Dreielektrodenanordnung auf, die mit einer gepulsten Spannung angesteuert wird (siehe dazu beispielsweise EP-OS 0 170 998). Dazu werden der Meß- bzw. Sensorelektrode zyklisch zwei Potentiale aufgeprägt, nämlich ein Meßpotential und ein sogenanntes Erholungspotential. Die Verweildauer beim Meßpotential ist dabei klein im Vergleich zur Zyklusdauer; auf diese Weise wird der Sauerstoffverbrauch sehr niedrig gehalten. Als Meßsignal wird der während der Meßperiode fließende Strom ausgewertet, wobei der Strom vorteilhaft integriert und das Ladungssignal ausgewertet wird; das Integral des Stromes wird dabei vorzugsweise mit einer Zeitverzögerung gebildet. Die ermittelte Ladung ist dann ein Maß für die Sauerstoffkonzentration. Bei einer derartigen Impulsmethode liegt der Leistungsbedarf unterhalb 50 µW. Der erfindungsgemäße Sensor ist deshalb auch als Langzeitimplantat einsetzbar.

Der elektrochemisch-enzymatische Sensor nach der Erfindung ist relativ einfach aufgebaut. Neben der eigentlichen Meßelektrode, die auch als Arbeitselektrode bezeichnet wird, und einer Gegen- und Bezugselektrode sowie einem Enzym erfordert er lediglich eine Membran. Bei den bekannten Enzymsensoren dagegen werden meistens zwei Membranen verwendet. Eine Membran dient dabei dazu, das Enzym vom umgebenden Elektrolyten abzutrennen, die andere Membran wird unmittelbar vor der Meßelektrode angeordnet. Diese Membran ist meistens nur für eine bestimmte Molekülart durchlässig, damit eine möglichst spezifische Elektrodenreaktion erhalten wird. Für einen derartigen Zweck wird häufig eine sogenannte Carrier-PVC-Membran eingesetzt, die aber bei der Anwendung in Implantaten aufgrund der Toxizität der enthaltenen Komponenten problematisch ist.

Die beim erfindungsgemäßen Sensor eingesetzte Membran besteht vorzugsweise aus Celluloseacetat oder perfluorsulfoniertem Polytetrafluorethylen; es kann beispielsweise aber auch Polyurethan verwendet werden. Allgemein ist diese Membran, die einerseits das Enzym zurückhält und andererseits durchlässig für Sauerstoff ist, biokompatibel und hydrophil, d.h. durchlässig für die zu bestimmende Substanz. Aufgrund der hohen Empfindlichkeit des Sensors für Sauerstoff kann dabei eine Membran gewählt werden, die für die zu bestimmende Substanz, wie Glucose, einen niedrigen Diffusionskoeffizienten besitzt, beispielsweise unterhalb 10⁻¹² m²/s. Der Diffusionskoeffizient für Sauerstoff soll derart sein, daß einerseits genügend Sauerstoff für die Enzymreaktion zur Verfügung steht, andererseits aber kein Überschuß vorhanden ist. Für die maximal meßbare Konzentration der zu bestimmenden Substanz ist das Verhältnis zwischen der Sauerstoffdiffusion und der Diffusion dieser Substanz entscheidend. Dieses Verhältnis wird durch den Charakter der Membran bestimmt, d.h. durch den Grad der Hydrophilität.

Die Dicke der Membran ist relativ unkritisch, da die Ansprechzeit des Sensors für Sauerstoff sehr kurz ist, für die eigentliche Messung (der jeweiligen Substanz) aber Ansprechzeiten von einigen Minuten tolerierbar sind. Im allgemeinen beträgt die Membrandicke 10 bis 150 µm.

Als Material für die Sensorelektrode dient vorteilhaft Glaskohlenstoff, Pyrographit, gesputterter Kohlenstoff, gesputterter Graphit und amorpher wasserstoffhaltiger Kohlenstoff (a-C:H); vorzugsweise wird dabei Glaskohlenstoff eingesetzt, und zwar in nicht-aktivierter Form, d.h. es wird eine sogenannte glatte Glaskohlenstoffelektrode verwendet. Die Gegenelektrode besteht vorteilhaft aus Platin oder aktiviertem Glaskohlenstoff. Die Bezugselektrode ist vorzugsweise eine Ag/AgCl-Elektrode.

Für Implantationszwecke dient vorteilhaft eine Sensoranordnung mit zwei Meßelektroden, von denen eine als Sensorelektrode mit Enzym, die andere als Sensorelektrode ohne Enzym ausgestaltet ist, d.h. im zweiten Fall liegt beispielsweise eine Lösung vor, die kein Enzym enthält. Dabei wird bei der Messung von Impuls zu Impuls von einer Elektrode zur anderen umgeschaltet; die Differenz der dabei erhaltenen Signale entspricht der Konzentration der zu bestimmenden Substanz. Bei einer derartigen Anordnung, die sich gut zur Langzeitimplantation eignet, weil eine Drift des Meßsignals ausgeschaltet ist, weisen die beiden Sensoren, d.h. die beiden Meßelektroden, vorzugsweise zusammen nur eine Gegenelektrode und eine Bezugselektrode auf. Derartige für Implantationszwecke geeignete Anordnungen werden vorteilhaft als Scheibensensoren ausgebildet. Bei Messungen im Blut werden dagegen vorzugsweise sogenannte Tip-Elektroden eingesetzt. Bei derartigen Elektroden wird auf die Elektrodenspitze (der Meßelektrode) beispielsweise eine ein Enzym enthaltende Polymerlösung aufgebracht und nach dem Trocknen mit einer Membranschicht abgedeckt.

Anhand von Ausführungsbeispielen soll die Erfindung noch näher erläutert werden.

Für die Untersuchungen werden Durchflußmeßzellen aus Polysulfon eingesetzt, wobei eine glatte Glaskohlenstoffelektrode als Arbeitselektrode dient (Durchmesser: 5 mm; Fläche: 0,2 cm²). Die Bezugselektrode ist eine Ag/AgCl-Elektrode, als Gegenelektrode dient eine Platinelektrode (sandgestrahlt) oder eine Elektrode aus aktiviertem Glaskohlenstoff (Fläche jeweils 0,2 cm²).

Zur Konzentrationsbestimmung von Glucose dient beispielsweise ein Sensor, bei dem sich im Raum zwischen der Arbeitselektrode und einer Membran aus Nafion, d.h. perfluorsulfoniertem Polytetrafluorethylen (Dicke: ca. 35 µm; Diffusionskoeffizient für Glucose: 5,9.10⁻¹³ m²/s, für O₂: 20.10⁻¹³ m²/s), 10 µl einer wäßrigen Lösung von Glucoseoxidase (in physiologischer Kochsalzlösung oder Ringerlösung) befinden. Als Elektrolyt dient eine Ringerlösung mit einem Gehalt von 10 % O₂. Der Sensor wird nach dem Impulsverfahren betrieben (Impulsdauer: 20 ms, Integration: 15 bis 20 ms, Zyklusdauer: 2 s).

Bei 25°C werden gut reproduzierbare Meßkurven erhalten. Dabei ist die Abnahme des Sensorsignals mit der Zeit wesentlich geringer als bei herkömmlichen Sensoren. Dies ist darauf zurückzuführen, daß die Sensorelektrode nicht vergiftet wird. Vergleichbare Ergebnisse ergeben sich auch bei 37°C in Ringerlösung. Auch bei der Verwendung von defibriniertem Schafblut als Elektrolyt werden zufriedenstellende Ergebnisse erzielt.

Wird eine dickere, d.h. weniger hydrophile Membran eingesetzt (Dicke: ca. 105 µm), so ist die Empfindlichkeit höher, weil die Sauerstoffdiffusion - gegenüber einer verminderten Glucosediffusion - verbessert ist (Diffusionskoeffizient für Glucose: 3,1.10⁻¹³ m²/s, für O₂: 14.10⁻¹³ m²/s). Derartige Sensoren wurden im Dauerversuch (bei 25°C) über einen Zeitraum von mehr als 100 Tagen erfolgreich getestet.

Bei der Bestimmung von Lactat beispielsweise befindet sich in entsprechender Weise eine Lösung von Lactatoxidase (10 µl) direkt vor der Arbeitselektrode. Die Membran besteht in diesem Fall beispielsweise aus Celluloseacetat oder -nitrat.

Der Sensor nach der Erfindung kann auch in Form eines Kathetersensors aufgebaut sein. Zur Bestimmung von Glucose beispielsweise dient hierbei eine Dreielektrodenanordnung mit einer Glaskohlenstoffelektrode als Spitze und einer dahinter isoliert angeordneten Ag/AgCl-Bezugselektrode; isoliert dahinter folgt dann eine Pt-Gegenelektrode. Auf die Arbeitselektrode aus Glaskohlenstoff wird das Enzym Glucoseoxidase in der Weise aufgebracht, daß die Elektrode in ein Gemisch aus Glucoseoxidaselösung, Rinderserumalbumin und Glutaraldehydlösung (5 %) eingetaucht wird; anschließend wird getrocknet. Nachfolgend wird der Katheter, einschließlich der Bezugselektrode, in eine Nafionlösung (5 %, Isopropanol) eingetaucht, um die Enzymschicht mit einer hydrophilen Membran abzudecken. Eine derartige Membran wird beispielsweise auch mittels einer Lösung von Polyurethan (10 %, N-Methylpyrrolidon) erhalten.

## Patentansprüche

1. Elektrochemisch-enzymatischer Sensor zur Bestimmung von Substanzen, insbesondere Glucose, in Körperflüssigkeiten, **gekennzeichnet** durch
- eine Sensorelektrode aus elektrokatalytisch inaktivem Kohlenstoff in Form von Glaskohlenstoff, Pyrographit, gesputtertem Kohlenstoff, gesputtertem Graphit oder amorphem wasserstoffhaltigem Kohlenstoff, an der nur Sauerstoff umgesetzt wird
- eine Gegenelektrode
- eine Bezugselektrode
- eine vor der Sensorelektrode befindliche Enzym enthaltende Schicht und
- eine die Enzymschicht zur Körperflüssigkeit hin abdeckende und das Enzym zurückhaltende Membran aus biokompatiblem, hydrophilem, sauerstoffdurchlässigem Material.

2. Sensor nach Anspruch 1, **dadurch gekennzeichnet**, daß die Gegenelektrode aus Platin oder aktiviertem Glaskohlenstoff besteht.

3. Sensor nach Anspruch 1 oder 2, **dadurch gekennzeichnet**, daß die Bezugselektrode eine Ag/AgCl-Elektrode ist.

4. Sensor nach einem der Ansprüche 1 bis 3, **dadurch gekennzeichnet,** daß das Enzym in gelöster oder immobilisierter Form vorliegt.

5. Sensor nach einem oder mehreren der Ansprüche 1 bis 4, **dadurch gekennzeichnet**, daß die Membran aus Celluloseacetat oder perfluorsulfoniertem Polytetrafluorethylen besteht.

6. Implantierbare Sensoranordnung, **gekennzeichnet** durch einen ersten Sensor nach einem oder mehreren der Ansprüche 1 bis 5 und einen zweiten Sensor mit entsprechendem Aufbau, aber ohne Enzym.

7. Sensoranordnung nach Anspruch 6, **dadurch gekennzeichnet**, daß die beiden Sensoren zusammen nur eine Gegenelektrode und eine Bezugselektrode aufweisen.

## Claims

1. Electrochemical enzymatic sensor for determining substances, in particular glucose, in body fluids, **characterised by**
- a sensor electrode of electrocatalytically inactive carbon in the form of vitreous carbon, pyrographite, sputtered carbon, sputtered graphite or amorphous hydrogenated carbon, at which only oxygen is converted,
- a counterelectrode,
- a reference electrode,
- an enzyme-containing layer located before the sensor electrode, and
- a diaphragm of biocompatible, hydrophilic, oxygen-permeable material covering the enzyme layer towards the body fluid and retaining the enzyme.

2. Sensor according to claim 1, **characterised in that** the counterelectrode consists of platinum or activated vitreous carbon.

3. Sensor according to claim 1 or 2, **characterised in that** the reference electrode is an Ag/AgCl electrode.

4. Sensor according to one of claims 1 to 3, **characterised in that** the enzyme is present in dissolved or immobilised form.

5. Sensor according to one or more of claims 1 to 4, **characterised in that** the diaphragm consists of cellulose acetate or perfluorosulfonated polytetrafluoroethylene.

6. Implantable sensor arrangement, **characterised by** a first sensor according to one or more of claims 1 to 5 and a second sensor having a structure corresponding to the first sensor, but without enzyme.

7. Sensor arrangement according to claim 6, **characterised in that** the two sensors jointly have only one counterelectrode and one reference electrode.

## Revendications

1. Senseur électrochimique enzymatique pour la détermination de substances, en particulier du glucose, dans des liquides corporels, caractérisé par
- une électrode de détection en carbone électrocatalytiquement inactif sous forme de carbone vitreux, de pyrographite, de carbone pulvérisé cathotiquement, de graphite pulvérisé cathotiquement ou de carbone amorphe contenant de l'hydrogène, sur laquelle ne réagit que l'oxygène
- une contre-électrode
- une électrode de référence
- une couche contenant une enzyme, se trouvant devant l'électrode de détection et
- une membrane recouvrant la couche d'enzyme du côté du liquide corporel et retenant l'enzyme, constituée d'un matériau biocompatible, hydrophile et perméable à l'oxygène.

2. Senseur selon la revendication 1, caractérisé en ce que la contre-électrode est constituée de platine ou de carbone vitreux activé.

3. Senseur selon la revendication 1 ou 2, caractérisé en ce que l'électrode de référence est une électrode de type Ag/AgCl.

4. Senseur selon l'une quelconque des revendications 1 à 3, caractérisé en ce que l'enzyme se trouve sous forme solubilisée ou immobilisée.

5. Senseur selon une ou plusieurs des revendications 1 à 4, caractérisé en ce que la membrane est constituée d'acétate de cellulose ou de polytétrafluoroéthylène perfluorosulfoné.

6. Dispositif de senseur implantable, caractérisé par un premier senseur selon une ou plusieurs des revendications 1 à 5 et par un deuxième senseur avec une structure correspondante, mais sans enzyme.

7. Dispositif de senseur selon la revendication 6, caractérisé en ce que les deux senseurs ensemble ne présentent qu'une contre-électrode et qu'une électrode de référence.
